# EUROPEAN PATENT APPLICATION

(11) **EP 3 757 227 A1**
(43) Date of publication of application: **30.12.2020**
(21) Application number: 19182233.7
(22) Date of filing: 25.06.2019
(51) Int. Cl.: C12Q 1/6806, G01N 33/574

(54) **CELL-FREE CHROMATIN IMMUNOPRECIPITATION (CFCHIP) AS A MEASURE OF TUMOUR GENE EXPRESSION IN A SAMPLE**

(71) Applicant: Aarhus Universitet, 8000 Aarhus C (DK)
(72) Inventor: Vad-Nielsen, Johan, 8000 Aarhus C (DK); Lade-Nielsen, Anders, 8230 Åbyhøj (DK); Sandahl Sørensen, Boe, 8330 Beder (DK)
(74) Representative: Plougmann Vingtoft a/s

(57) **Abstract**

The present invention relates to a method of subtyping a cancer, staging a cancer, or determining the risk of developing cancer for an individual, said method comprising the steps of 1) contacting a biological sample from said individual with an antibody that binds to a histone; 2) isolating nucleosomes and/or histones associated to said antibody; 3) identifying at least one gene associated with the isolated nucleosome and/or histone, to identify and /or quantify the expression level of a gene in said sample from said individual; 4) determining a subtype of a cancer, staging a cancer, and/or a risk of developing cancer for said individual, based on the identification of nucleosome and/or histone associated gene(s) in said sample.

SEQUENCE LISTING

<110>  Aarhus University

<120>  Cell-free Chromatin Immunoprecipitation (cfChIP) as a measure of
       tumour gene expression in a sample

<130>  68218EP01

<160>  19

<170>  PatentIn version 3.5

<210>  1
<211>  20
<212>  DNA
<213>  Artificial Sequence

<220>
<223>  Primer

<400>  1
gctgttccag gctctgttcc                                                   20


<210>  2
<211>  19
<212>  DNA
<213>  Artificial Sequence

<220>
<223>  Primer

<400>  2
gctcacacgc cacaacatg                                                    19


<210>  3
<211>  20
<212>  DNA
<213>  Artificial Sequence

<220>
<223>  Primer

<400>  3
cagcataggc caagtacacg                                                   20


<210>  4
<211>  20
<212>  DNA
<213>  Artificial Sequence

<220>
<223>  Primer

<400>  4
tattttcttc cagccccagg                                                   20


<210>  5
<211>  20

<212>  DNA
<213>  Artificial Sequence

<220>
<223>  Primer

<400>  5
tcatccaacg gaagctaatg                                                    20


<210>  6
<211>  20
<212>  DNA
<213>  Artificial Sequence

<220>
<223>  Primer

<400>  6
cgtttcaatt cgatggtgtt                                                    20


<210>  7
<211>  18
<212>  DNA
<213>  Artificial Sequence

<220>
<223>  Primer

<400>  7
gtttctttcg ctgttcca                                                      18


<210>  8
<211>  17
<212>  DNA
<213>  Artificial Sequence

<220>
<223>  Primer

<400>  8
gcaggcagaa accaaat                                                       17


<210>  9
<211>  26
<212>  DNA
<213>  Artificial Sequence

<220>
<223>  Probe


<220>
<221>  misc_feature
<222>  (1)..(1)
<223>  HEX modification

<220>
<221>  misc_feature
<222>  (26)..(26)

<223>  BHQ1 modification

<400>  9
cccggcattt cctccctgaa gcctcc                                          26


<210>  10
<211>  19
<212>  DNA
<213>  Artificial Sequence

<220>
<223>  Primer

<400>  10
ctgaggctga gtcctggta                                                  19


<210>  11
<211>  17
<212>  DNA
<213>  Artificial Sequence

<220>
<223>  Primer

<400>  11
aagtctgcag tcctctg                                                    17


<210>  12
<211>  18
<212>  DNA
<213>  Artificial Sequence

<220>
<223>  Primer

<400>  12
ctgaggtcaa ggcccaat                                                   18


<210>  13
<211>  20
<212>  DNA
<213>  Artificial Sequence

<220>
<223>  Primer

<400>  13
cggtggatct cagcaatctc                                                 20


<210>  14
<211>  19
<212>  DNA
<213>  Artificial Sequence

<220>
<223>  Primer

<400>  14

ctgaggctga gtcctggta                                                    19

<210>  15
<211>  17
<212>  DNA
<213>  Artificial Sequence

<220>
<223>  Primer

<400>  15
aagtctgcag tcctctg                                                      17

<210>  16
<211>  21
<212>  DNA
<213>  Artificial Sequence

<220>
<223>  Probe

<220>
<221>  misc_feature
<222>  (1)..(1)
<223>  FAM modification

<220>
<221>  misc_feature
<222>  (21)..(21)
<223>  BHQ1 modification

<400>  16
agcagggagt gggcagccgc t                                                 21

<210>  17
<211>  20
<212>  DNA
<213>  Artificial Sequence

<220>
<223>  Primer

<400>  17
tgtgggtggg tgtgtctata                                                   20

<210>  18
<211>  20
<212>  DNA
<213>  Artificial Sequence

<220>
<223>  Primer

<400>  18
atttcccata gcagcactcc                                                   20

<210>  19

<211>  26
<212>  DNA
<213>  Artificial Sequence

<220>
<223>  Probe



<220>
<221>  misc_feature
<222>  (1)..(1)
<223>  FAM modification

<220>
<221>  misc_feature
<222>  (26)..(26)
<223>  BHQ1 modification

<400>  19
tgtcctctgt cccatgccca ggtcct                                          26

## Description

### Technical field of the invention

The present invention relates to a method for subtyping and/or staging a cancer. In particular, the present invention relates to a method for subtyping and/or staging a cancer using cell-free chromatin immunoprecipitation as a measure of tumour gene expression in a sample.

### Background of the invention

Lung cancer (LC) is the most frequent cause of cancer related death among men and in some countries among women as well. The prognosis is poor and the overall 5-year survival is 8-10%. Non-small cell LC (NSCLC) accounts for 80-85% of the primary LC and includes the histological subtype's adenocarcinomas (LADC), squamous cell carcinomas (LSCC), and large cell carcinoma. At the time of diagnosis, the disease is often advanced; only 20% is operable, leading to a poor overall survival. This is due to the lack of eligible screening and early symptoms, an insufficient drug repertoire, and an urgent request for additional molecular biomarkers for diagnosis and treatment stratification. At present, the NSCLC examination program includes imaging and invasive procedures like endoscopy taken tissue biopsies. These are used to determine the tumor histological type as well as the genetic characteristics e.g. presence of driver EGFR, KRAS, or ALK gene mutations and gene expression profiling, with a resulting stratification of patients to achieve the most optimal treatment at the given cancer stage.

In a tumor, gene-expression is a dynamic process, allowing the cancer cells to adapt rapidly to environmental or physiological changes. Thus, gene-expression profiling can be a powerful way to identify gene-expression biomarkers with diagnostic capability for survey of i.e. cancer type, stage, and treatment response. To perform tumor gene-expression profiling and for gene-expression based biomarker analyses, a tumor tissue biopsy is currently required. However, tissue biopsies are not always available due to the anatomical location of the tumor and obtaining a tissue biopsy can even be associated with increased morbidity due to post-biopsy infections. Thus, tissue biopsies often are taken only once (at the time of diagnosis) and are not available for long-term post-diagnostic workup and monitoring. In addition, tissue biopsies only yield information regarding that particular tumor site which can be misleading due to the high degree of intra-tumor heterogeneity and metastasis in advanced tumors.

Non-invasive diagnostic methods with the ability to detect LC at an earlier stage (possibly screening) and securing optimal patient diagnosis and treatment stratification would be largely beneficial to improve the prognosis. The presence of cell-free tumour DNA in the blood of cancer patients has in recent years represented an attractive alternative to tumour biopsies for obtaining relevant tumour/cancer-material for molecular analyses and have enabled the possibility of longitudinal studies of i.e. cancer progression and treatment resistance development. This has allowed efficient description of mutational status by DNA sequencing including identifying oncogenic-drivers and quantitative measurements of copy-number variations. Moreover, DNA methylation analyses of circulating tumour DNA has been shown to represent a potential epigenetic based biomarker with possibility to describe gene expression deregulation for genes having relevance for cancer diagnosis, prognosis, and treatment selection.

However, since DNA methylation analyses only have informative importance for a limited subset of the genes, the need is urgent for alternative technology approaches.

Hence, an improved method for staging/subtyping a cancer would be advantageous, and in particular a more efficient and/or reliable non-invasive method would be advantageous.

### Summary of the invention

In here a method called Cell-Free Chromatin ImmunoPrecipitation (cfChIP) is disclosed, which can be used as a molecular technology to describe the expression status for in principle any gene in a tumor/cancer-cell population (i.e. primary tumor as well as metastatic tumors) using a liquid biopsy (i.e. a blood sample). cfChIP may have major implications for improved diagnostics, prognostics, and treatment in future precision oncology.

cfChIP methodology indirectly quantifying how genes are expressed in a solid tumor using blood plasma from a cancer patient. The experimental background of a cfChIP is the quantification of nucleosome modifications located at sequence-specific loci correlated to gene-expression and originating from the solid tumor but now present in e.g. the blood, but also other body liquids, of the cancer patient. Phrased in another way, cfChIP can be used as a measure of e.g. tumour gene expression using a liquid biopsy.

To achieve improved medical care of the individual cancer patient, cfChIP can be a preferred biomarker methodology, since it will allow a longitudinal characterization and monitoring of cancer subtype, progression, and relapse in response to treatment. Unlike current biopsy-based standards for quantifying tumor gene-expression, a cfChIP only requires a blood sample, or other liquid body fluid from the cancer patient, and thus constitutes a non-invasive and non-harmful procedure for the cancer patient. Moreover, due to relatively simple experimental requirements, usage of cfChIP can be feasible in clinical settings. cfChIP assays can be used for the wide range of cancer diagnostic applications wherein knowledge concerning the gene-expression profile in the solid tumor is of major importance in decisions regarding treatment strategy. cfChIP biomarker assays, can in the clinic will be supportive, or even alternative, to existing methodologies to securing improved, and personalized, cancer diagnosis, prognosis, and treatment.

For instance, Example 1 outlines the different steps in the method.

Example 4 discloses that using the method of the invention it is possible to discriminate (subtyping or staging) between LSCC and LADC lung cancers using a blood plasma sample. The diagnosis of LSCC versus LADC has major impact for patient prognosis and selection of treatment protocol. Noticeable, initially diagnosed LSCC or LADC will in later cancer stages sometimes be reverted to the other subtype. No known DNA mutations can readily distinguish between LADC and LSCC hindering use of liquid biopsies for the distinguishing of these cancer subtypes, and accordingly the cancer subtype is for the time being determined by immunohistochemical analysis on tissue biopsies. This is a relative time-consuming procedure to obtain the correct diagnosis of cancer subtype. Moreover, tissue biopsies are not always available due to the anatomical location of the tumor and obtaining a tissue biopsy can even be associated with increased morbidity due to post-biopsy infections. Thus, tissue biopsies often are taken only once (at the time of diagnosis) and are not available for long-term post-diagnostic workup and monitoring. In addition, tissue biopsies only yield information regarding that particular tumor site which can be misleading due to the high degree of intra-tumor heterogeneity and metastasis in advanced tumors.

Further, example 5 provides data showing that *PD-L1* serves as a gene of interest in the method of the invention for the improved diagnosis and prognosis of NSCLC patients in evaluating the eligibility for immunotherapy.

Examples 6-9 shows examples of other relevant genes to analyse in the method of the invention.

Thus, an object of the present invention relates to the provision of an improved method for staging/subtyping a cancer, and in particular to a more efficient and/or reliable non-invasive method.

Thus, one aspect of the invention relates to a method of subtyping a cancer, staging a cancer, or determining the risk of developing cancer for an individual, said method comprising the steps of
a) contacting a biological sample from said individual with an antibody or antibody fragment that binds to a nucleosome and/or histone;
b) isolating nucleosomes and/or histones associated to said antibody;
c) optionally, purifying DNA associated with said nucleosomes and/or histones;
d) identifying and/or quantifying at least one gene or part of gene associated with the isolated nucleosome and/or histone or present in the optionally purified DNA, to identify and/or quantify the level of expression of a gene in said sample from said individual;
e) optionally, comparing said identified and/or quantified expression level of the at least one gene to one or more reference levels; and
f) determining a subtype of a cancer, staging a cancer, and/or a risk of developing cancer for said individual, based on the expression level of the one or more genes.

Another aspect of the present invention relates to a kit of parts comprising
- a first container comprising an antibody (or other binding moiety) against a histone modification, said histone being modified indicative of being associated with an expressed or repressed gene;
- a second container comprising one or more, preferably two primers, for a gene of interest, preferably the gene is a *KRT6* gene, more preferably *KRT6ABC*; and
- optionally, one or more further containers comprising primer sets for one or more further genes of interest, such as one or more genes functioning as positive or negative controls for gene expression;
- optionally, one or more containers comprising components for initiating a PCR reaction using said primers;
- optionally, one or more probes for detecting a PCR product; and
- optionally, instructions for using the kit in a method according to the invention.

The present invention may have different uses. In an aspect, the invention relates to the use of a kit according to the invention for determining a subtype of a cancer, staging a cancer, and/or a risk of developing cancer.

In another aspect, the invention relates to the use of genes or part of genes associated with nucleosomes comprising histones, said histones being indicative of being associated with an expressed gene or a repressed gene for determining a subtype of a cancer, staging a cancer, and/or a risk of developing cancer.

### Brief description of the figures

Figure 1 shows an overview of the basics for the invention. A) H3K36me3 occupancy correlates to gene transcription. B) cfChIP infers gene expression from a blood plasma sample.
Figure 2 shows that using *in vitro* grown NSCLC cell lines (n=4) for ChIP the addressed genes *ACTG1*, *ALK*, and *SAT2* shows the expected ChIP result with *ACTG1* assigned an active gene and *ALK* and *SAT2* assigned inactive gene sequences.
Figure 3 shows that using NSCLC patient plasma samples (n=5) for cfChIP the same results are obtained as using *in vitro* grown NSCLC cell lines as described in figure 2.
Figure 4 shows that indirect H3K36me3-based ChIP analyses of *KRT6ABC* expression status relative to *ACTG1*, *ALK*, and *SAT2* expression status can distinguish NSCLC adenocarcinoma (LADC) from NSCLC squamous carcinoma (LSCC) biological samples with ChIP using *in vitro* cultured NSCLC cell lines (panels A and B) and cfChIP using NSCLC patient blood plasma samples (panels C and D).
Figure 5 shows that *PD-L1* (*CD274*) expression at the mRNA level correlates with H3K36me3 occupancy measured by ChIP in two NSCLC cell lines (HCC827 and HCC827-ER).
Figure 6 shows that *EGFR* (epidermal growth factor receptor, also abbreviated *HER1* and *ERbB1*) expression at the mRNA level correlates with H3K36me3 occupancy measured by ChIP in two NSCLC cell lines (HCC827 and HCC827-ER).
Figure 7 shows that expression at the mRNA level of epithelial to mesenchymal transition (EMT) marker genes correlate with H3K36me3 occupancy measured by ChIP in two NSCLC cell lines (HCC827 and HCC827-ER).

The present invention will now be described in more detail in the following.

### Detailed description of the invention

### Definitions

Prior to discussing the present invention in further details, the following terms and conventions will first be defined:

### Nucleosome

A protein/DNA complex composed of DNA and histone proteins (H2A, H2B, H3, H4, and variants of these). The histone proteins in a nucleosome can be post-translational modified. Whereas a "standard" nucleosome includes DNA and two units of each H2A, H2B, H3, H4 (together the eight proteins abbreviated a histone octamere) it will also be possible to have a stable complex between DNA and only a limited number of the histones (histone/DNA interactions). For the latter the histone proteins also can be post-translational modified.

### Chromatin immunoprecipitation (ChIP)

Isolation of nucleosomes/histones and associated DNA and/or RNA using one or several antibodies directed towards nucleosomes and/or histones either unmodified, posttranslational modified, or posttranslational modified in different combinations.

### Real-time quantitative PCR (qPCR)

A PCR reaction which quantifies the amount of starting DNA material in a given sample.

### Droplet Digital PCR (ddPCR)

In Digital Droplet PCR (ddPCR) the PCR sample is divided into smaller reactions through a water oil emulsion technique, which are then made to run PCR individually. Can quantify DNA in a given sample.

### Next generation sequencing (NGS)

Next generation sequencing (NGS, NextGenSeq) is a method for parallel sequencing DNA samples (E.g. genomes, cDNA genomes (RNA-seq), ChIP purified DNA) at high speed and at low cost. It is also known as second generation sequencing (SGS) or massively parallel sequencing (MPS).

### Nanostring nCounter

NanoString's nCounter technology is a variation on the DNA microarray. It uses molecular barcodes and microscopic imaging to detect and count (quantify) up to several hundred unique transcripts/DNA-fragments in one hybridization reaction.

### Non-small-cell lung carcinoma (NSCLC)

Non-small-cell lung carcinoma (NSCLC) is any type of epithelial lung cancer other than small cell lung cancer (SCLC). NSCLC accounts for about 85% of all lung cancers. As a class, NSCLCs are relatively insensitive to chemotherapy, compared to lung small cell cancer (SCLC). When possible, they are primarily treated by surgical resection with curative intent, although chemotherapy has been used increasingly both pre-operatively (neoadjuvant chemotherapy) and postoperatively (adjuvant chemotherapy). NSCLC has three major subtypes: adenocarcinoma (LADC) for 40%, squamous cell carcinoma (LSCC) for 30% and large cell carcinoma for 10%.

### Small cell lung cancer (SCLC)

About 10% to 15% of lung cancers are SCLC.

### Antibody

The term "antibody" as used herein refers to a protein of the immunoglobulin (Ig) superfamily that binds non-covalently to certain substances (antigens/analytes) to form an antibody-antigen/analyte complex. Antibodies can be endogenous, or polyclonal wherein an animal is immunized to elicit a polyclonal antibody response or by recombinant methods resulting in monoclonal antibodies produced from hybridoma cells or other cell lines. It is understood that the term "antibody" as used herein includes within its scope any of the various classes or sub-classes of immunoglobulin derived from any of the animals conventionally used.

### Antibody fragments

The term "antibody fragments" as used herein refers to fragments of antibodies that retain the principal selective binding characteristics of the whole antibody. Particular fragments are well-known in the art, for example, Fab, Fab', and F(ab')₂ which are obtained by digestion with various proteases, pepsin or papain, and which lack the Fc fragment of an intact antibody or the so-called "half-molecule" fragments obtained by reductive cleavage of the disulfide bonds connecting the heavy chain components in the intact antibody. Such fragments also include isolated fragments consisting of the light-chain-variable region, "Fv" fragments consisting of the variable regions of the heavy and light chains, and recombinant single chain polypeptide molecules in which light and heavy variable regions are connected by a peptide linker. Other examples of binding fragments include (i) the Fd fragment, consisting of the VH and CH1 domains; (ii) the dAb fragment, which consists of a VH domain; (iii) isolated CDR regions; and (iv) single-chain Fv molecules (scFv) described above. In addition, arbitrary fragments can be made using recombinant technology that retains antigen-recognition characteristics.

### Kit

The term "kit" as used herein refers to a packaged set of related components, typically one or more compounds or compositions.

### Reference level

In the context of the present invention, the term "reference level" relates to a standard in relation to a quantity, which other values or characteristics can be compared to.

In one embodiment of the present invention, it is possible to determine a reference level by investigating the abundance of one or more of the biomarkers according to the invention in samples from healthy subjects (in the present context e.g. patients without cancer).

In another embodiment of the present invention, it is possible to determine a reference level by investigating the abundance of one or more of the biomarkers according to the invention in samples from the same subject (e.g. obtained at previous time points).

By applying different statistical means, such as multivariate analysis, one or more reference levels can be calculated.

Based on these results, a cut-off may be obtained that shows the relationship between the level(s) detected and patients at risk. The cut-off can thereby be used to determine the amount of the one or more biomarkers, which corresponds to for instance an increased risk of a subject for having cancer.

### Risk Assessment

The present inventors have successfully developed a new method for subtyping a cancer, staging a cancer, or determining the risk of developing cancer for an individual. To e.g. determine whether a patient has an increased risk of developing cancer a cut-off must be established. This cut-off may be established by the laboratory, the physician or on a case-by-case basis for each patient.

The cut-off level could be established using a number of methods, including: multivariate statistical tests (such as partial least squares discriminant analysis (PLS-DA), random forest, support vector machine, etc.), percentiles, mean plus or minus standard deviation(s); median value; fold changes.

The multivariate discriminant analysis and other risk assessments can be performed on the free or commercially available computer statistical packages (SAS, SPSS, Matlab, R, etc.) or other statistical software packages or screening software known to those skilled in the art.

As obvious to one skilled in the art, in any of the embodiments discussed above, changing the risk cut-off level could change the results of the discriminant analysis for each subject.

Statistics enables evaluation of the significance of each level. Commonly used statistical tests applied to a data set include t-test, f-test or even more advanced tests and methods of comparing data. Using such a test or method enables the determination of whether two or more samples are significantly different or not.

The significance may be determined by the standard statistical methodology known by the person skilled in the art.

The chosen reference level may be changed depending on the subject for which the test is applied.

Preferably, the subject according to the invention is a human subject, such as a subject considered at risk of having delayed or slow graft function.

The chosen reference level may be changed if desired to give a different specificity or sensitivity as known in the art. Sensitivity and specificity are widely used statistics to describe and quantify how good and reliable a biomarker or a diagnostic test is. Sensitivity evaluates how good a biomarker or a diagnostic test is at detecting a disease, while specificity estimates how likely an individual (i.e. control, patient without disease) can be correctly identified as not at risk. Several terms are used along with the description of sensitivity and specificity; true positives (TP), true negatives (TN), false negatives (FN) and false positives (FP). If a disease is proven to be present in a sick patient, the result of the diagnostic test is considered to be TP. If a disease is not present in an individual (i.e. control, patient without disease), and the diagnostic test confirms the absence of disease, the test result is TN. If the diagnostic test indicates the presence of disease in an individual with no such disease, the test result is FP. Finally, if the diagnostic test indicates no presence of disease in a patient with disease, the test result is FN.

### Sensitivity

$Sensitivity = TP / \left(TP+FN\right) = number of true positive assessments / number of all samples from patients with disease .$

As used herein, the sensitivity refers to the measures of the proportion of actual positives, which are correctly identified as such.

### Specificity

$Specificity = TN / \left(TN+FP\right) = number of true negative assessments / number of all samples from controls .$

As used herein, the specificity refers to measures of the proportion of negatives, which are correctly identified. The relationship between both sensitivity and specificity can be assessed by the ROC curve. This graphical representation helps to decide the optimal model through determining the best threshold -or cut-off for a diagnostic test or a biomarker candidate.

As will be generally understood by those skilled in the art, methods for screening are processes of decision-making and therefore the chosen specificity and sensitivity depend on what is considered to be the optimal outcome by a given institution/clinical personnel.

It would be obvious for a person skilled in the art that it may be advantageous to select a higher sensitivity at the expense of lower specificity in most cases, to identify as many patients with disease risk as possible.

In a preferred embodiment, the invention relates to a method with a high specificity, such as at least 70%, such as at least 80%, such as at least 90%, such as at least 95%, such as 100%. In another preferred embodiment, the invention relates to a method with a high sensitivity, such as at least 80%, such as at least 90%, such as 100%.

### Method of subtyping a cancer, staging a cancer, or determining the risk of

### developing cancer for an individual

As outlined above, the present invention relates to a technology to describe the expression status for in principle any gene in a tumor/cancer-cell population (i.e. primary tumor as well as metastatic tumors) using a liquid biopsy (i.e. a blood sample). Thus, an aspect of the invention relates to a method of subtyping a cancer, staging a cancer, or determining the risk of developing cancer for an individual, said method comprising the steps of
a) contacting a biological sample from said individual with an antibody or antibody fragment that binds to a nucleosome and/or histone;
b) isolating nucleosomes and/or histones associated to said antibody;
c) optionally, purifying DNA associated with said nucleosomes and/or histones;
d) identifying and/or quantifying at least one gene or part of gene associated with the isolated nucleosome and/or histone or present in the optionally purified DNA, to identify and/or quantify the level of expression of a gene in said sample from said individual;
e) optionally, comparing said identified and/or quantified expression level of the at least one gene to one or more reference levels; and
f) determining a subtype of a cancer, staging a cancer, and/or a risk of developing cancer for said individual, based on the expression level of the one or more genes.

As outlined in example 4, the method can e.g. be used for subtyping lung cancers.

Preferably, the method includes the step c) of purifying DNA associated with said nucleosomes and/or histones.

Preferably, the method includes the step e) of comparing said identified and/or quantified expression level of the at least one gene to one or more reference levels

The expression status of different genes may be determined by the method of the invention. Thus, in an embodiment, the gene is selected from the group consisting of a *KRT6* gene, such as *KRT6A*, *KRT6B*, *and KRT6C*, *ACTG1*, *ALK*, *SAT2*, *EGFR*, *hTERT*, *PD-L1*, *FGFR1*, *CDH1*, *VIM*, *ZEB1*, *KRT5*, *TP63*, *INSM1*, *NAPSA*, and *NKX2-*1 or combinations thereof, preferably *KRT6A*, *KRT6B*, and *KRT6C.* In example 4 the expression level of KRT6ABC is determined, in example 5 data for PD-L1 is provided, in example 6 data for EGFR is provided, in examples 7-9 the rationale behind determining EMT, and the expression level of *hTERT*, *NAPSA*, *NKX2-1*, *TP63*, and *INSM1* is provided.

In another embodiment, the gene is a *KRT6* gene such as *KRT6A*, *KRT6B* and/or *KRT6C* or combinations thereof, such as *KRT6ABC.* As outlined in example 4, primer sets have been developed encompassing *KRT6A*, *KRT6B*, and *KRT6C.*

The method of the invention may find use for different types of cancer. Thus, in an embodiment, the cancer is selected from the group consisting of lung cancer, such as Non-small-cell lung carcinoma (NSCLC), such as adenocarcinoma (LADC), squamous cell carcinoma (LSCC), and large cell carcinoma (LCC) and small cell lung cancer (SCLC).

In another embodiment, the cancer is selected from the group consisting of Astrocytomas, Breast Carcinomas, Cervical Carcinoma, Colorectal Adenocarcinoma, Ependymomas, Esophageal Carcinoma, Gastric Adenocarcinoma, Glioblastomas, Head and Neck Squamous Cell Carcinoma, Hepatocellular Carcinoma, Kidney Carcinomas, Leukemia, Lymphomas, Meningiomas, Multiple Myeloma, Ovarian Serous Adenocarcinoma, Pancreatic Ductal Adenocarcinoma, Prostate Adenocarcinoma, Sarcoma, Skin Cutaneous Melanoma, Testicular Cancer, Thyroid Papillary Carcinoma, Uterine Carcinomas and Uveal Melanoma.

In a preferred embodiment, the cancer is lung cancer. Preferably the method is for subtyping a lung cancer.

In another preferred embodiment, the staging and/or subtyping of a cancer is staging or subtyping of LADC and LSCC. Example 4 shows exactly such subtyping. Different controls may also be included in the assay. Thus, in an embodiment the level of *ACTG1*, *ALK* and/or *SAT2* is also determined. In example 2, it is verified that these genes may function as positive and negative controls.

Different types of biological samples may be used for the method of the invention. Thus, in an embodiment, said biological sample is selected from the group consisting of a blood sample, such as whole blood, blood plasma or blood serum, saliva, urine, CSF or a tissue sample, preferably a blood plasma sample. In example 4 blood plasma samples have been used.

The antibody (or other similar molecule) may target different histones. Thus, in an embodiment, the histone is a histone or modified histone considered to be associated with an expressed gene or a repressed gene, such as a constitutively expressed gene or a cell type specific expressed gene, or a constitutively repressed gene, or a cell type specific repressed gene, such as the histone being selected from the group consisting of H3K36me3, H3K36me2 and H3K36me1, preferably H3K36me3.

In an embodiment, the histone is a histone or modified histone from histone families/subfamilies including the shown in Table 1. In another embodiment, the histone is post-translational modified with a modification including the modifications shown table 1.

In yet another embodiment, the histone is post-translational modified with combinations of modifications including modifications shown in table 1.

**Table 1 - Examples of histones and histone modifications**

| **HF** | **HSF** | **(gene names)** | **MS** | **Mod** | **MN** | **Func.*** |
|---|---|---|---|---|---|---|
| H1 | H1F | H1F0, H1FNT, H1FOO, H1FX | Lys26 (K26) | Me | 1,2,3 | Rep |
| | H1H1 | HIST1H1A, HIST1H1B, HIST1H1C, HIST1H1D, HIST1H1E, HIST1H1T | Ser27 (S27) | P | | Act |
| H2A | H2AF | H2AFB1, H2AFB2, H2AFB3, H2AFJ, H2AFV, H2AFX, H2AFY, H2AFY2, H2AFZ | Lys5 (K5) | Ac | | Act |
| | | | Ser1 (S1) | P | | Rep |
| | | | Tyr57 (Y57) | P | | Act |
| | H2A1 | HIST1H2AA, HIST1H2AB, HIST1H2AC, HIST1H2AD, HIST1H2AE, HIST1H2AG, HIST1H2AI, HIST1H2AJ, HIST1H2AK, HIST1H2AL, HIST1H2AM | Thr120 (T120) | p | | Act |
| | | | Lys119 (K119) | Ub | | Rep |
| | H2A2 | HIST2H2AA3, HIST2H2AC | | | | |
| H2B | H2BF | H2BFM, H2BFS, H2BFWT | Lys5 (K5) | Ac | | Act/Re |
| | H2B1 | HIST1H2BA, HIST1H2BB, HIST1H2BC, HIST1H2BD, HIST1H2BE, HIST1H2BF, HIST1H2BG, HIST1H2BH, HIST1H2BI, HIST1H2BJ, HIST1H2BK, HIST1H2BL, HIST1H2BM, HIST1H2BN, HIST1H2BO | Lys12 (K12) | Ac | | P |
| | | | Lys15 (K15) | Ac | | Act |
| | | | Lys20 (K20) | Ac | | Act |
| | | | Tyr37 (Y37) | P | | Act |
| | | | Lys120 (K120) | Ub | | Rep Act |
| | H2B2 | HIST2H2BE | | | | |
| H3 | H3A1 | HIST1H3A, HIST1H3B, HIST1H3C, HIST1H3D, HIST1H3E, HIST1H3F, HIST1H3G, HIST1H3H, HIST1H3I, HIST1H3J | Lys4 (K4) | Ac | | Act |
| | | | Lys9 (K9) | Ac | | Act |
| | | | Lys14 (K14) | Ac | | Act |
| | | | Lys18 (K18) | Ac | | Act |
| | H3A2 | HIST2H3C | Lys23 (K23) | Ac | | Act |
| | H3A3 | HIST3H3 | Lys27 (K27) | Ac | | Act |
| | | | Lys56 (K56) | Ac | | Act |
| | | | Arg2 (R2) | Me | 1, 2 | Act/Re |
| | | | Lys4 (K4) | Me | 1,2,3 | P |
| | | | Arg8 (R8) | Me | 1, 2 | Act |
| | | | Lys9 (K9) | Me | 1,2,3 | Rep |
| | | | Lys14 (K14) | Me | 1,2,3 | Act/Re |
| | | | Arg17 (R17) | Me | 1, 2 | P |
| | | | Lys27 (K27) | Me | 1,2,3 | Rep |
| | | | Lys36 (K36) | Me | 1,2,3 | Act |
| | | | Lys79 (K79) | Me | 1,2,3 | Act/Re |
| | | | Ser10 (S10) | P | | P |
| | | | Thr11 (T11) | P | | Act |
| | | | Ser28 (S28) | P | | Act/Re |
| | | | Arg2 (R2) | Ci | | P |
| | | | Arg8 (R8) | Ci | | Act |
| | | | Arg17 (R17) | Ci | | Act/Re |
| | | | Arg26 (R26) | Ci | | P |
| | | | Arg36 (R36) | Ci | | Act |
| | | | | | | Act |
| | | | | | | Act |
| | | | | | | Act |
| | | | | | | Act |
| | | | | | | Act |
| H4 | H41 | HIST1H4A, HIST1H4B, HIST1H4C, HIST1H4D, HIST1H4E, HIST1H4F, HIST1H4G, HIST1H4H, HIST1H4I, HIST1H4J, HIST1H4K, HIST1H4L | Lys5 (K5) | Ac | | Act |
| | | | Lys8 (K8) | Ac | | Act |
| | | | Lys12 (K12) | Ac | | Act |
| | | | Lys16 (K16) | Ac | | Act |
| | H44 | HIST4H4 | Arg3 (R3) | Me | 1, 2 | Act |
| | | | Lys20 (K20) | Me | 1,2,3 | Act |
| | | | Lys20 (K20) | Me | 1,2,3 | Act/Re |
| | | | Lys59 (K59) | Me | 1,2,3 | p |
| | | | Tyr88 (Y88) | P | | Rep |
| | | | Arg3 (R3) | Ci | | Act |
| | | | | | | Act |

HF: Histone family. HSF: Histone subfamily. MF: Modified site. Mod: Modification (Me: Methylation, P: Phosphorylation, Ac: Acetylation, Ub: Ubiquitination, and Ci: Citrullination. MN: Methylation number. Func*: Function: Gene regulatory function of modification*. Rep: Repression. Act: Activation.

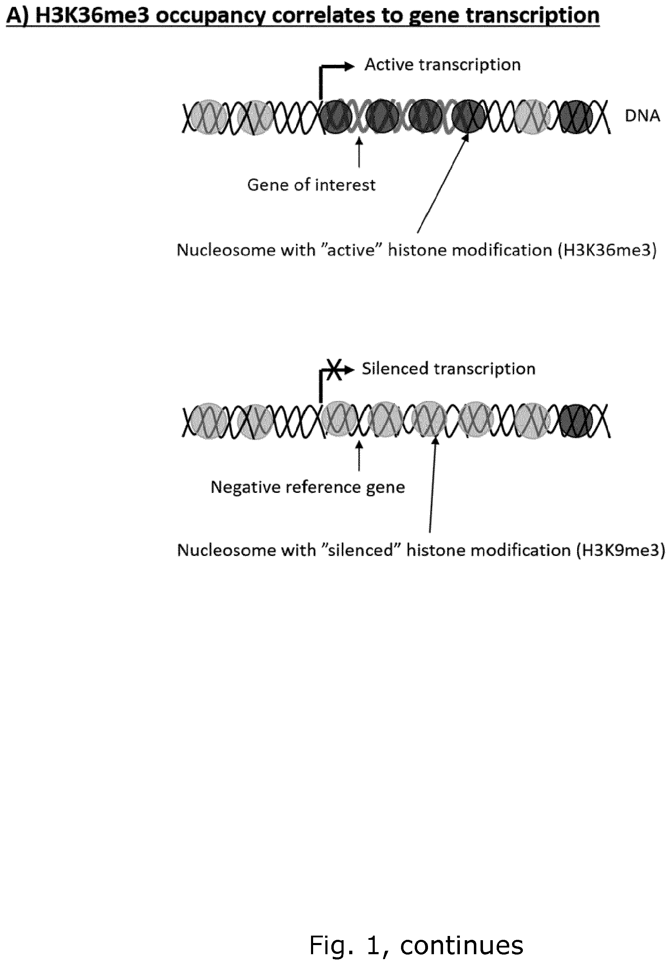

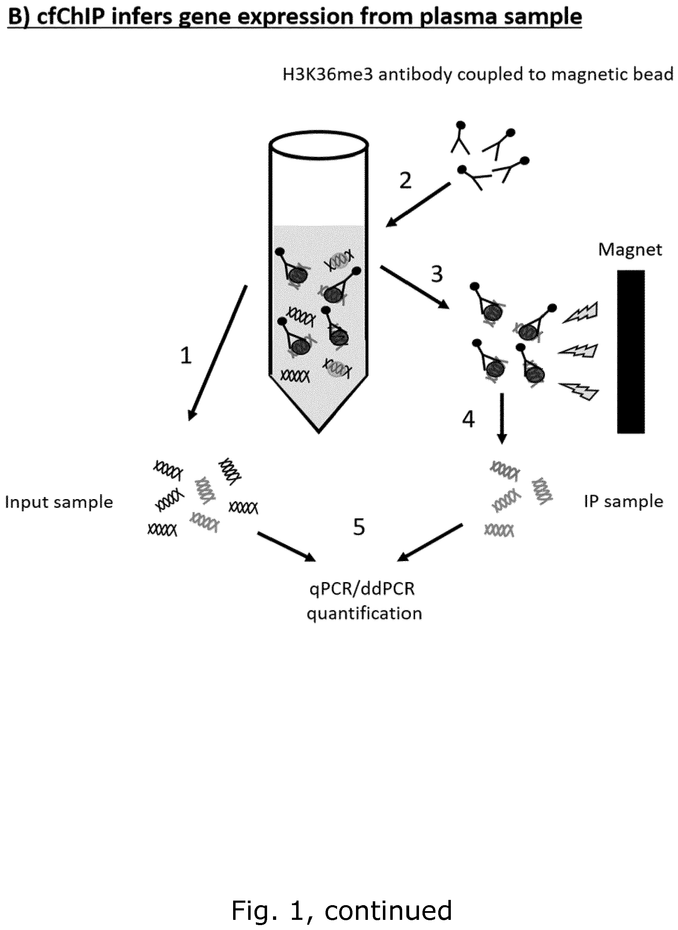

In an embodiment, the step c) of purifying said DNA associated with said nucleosomes, is performed by magnetic beads, Sepharose beads, and/or agarose beads. In figure 1 the method is outlined using magnetic beads, but the skilled person could find other methods.

The identification of the gene could be identified using different technologies. In an embodiment, the at least one gene is identified and/or quantified by PCR based technologies, such as qPCR or ddPCR, next generation sequencing, and/or Nanostring nCounter, preferably ddPCR.

In yet an embodiment a determined expression level of *KRT6,* preferably *KRT6ABC,* above said reference level is indicative of a lung cancer being a LSCC; whereas an expression level of *KRT6*, preferably *KRT6ABC*, equal to or below said reference level, is indicative of a lung cancer being a LADC. Again, example 4 provides data on subtyping based on *KRT6* expression.

In another embodiment, a determined expression level of *KRT6ABC* above said reference level is indicative of the NSCLC subtype being LSCC; whereas an expression level of *KRT6ABC* equal to or below said reference is indicative of the NSCLC subtype level being LADC. Selection of therapy eligibility of NSCLC patients depend on diagnosed LSCC or LADC.

Targeted therapy is more commonly available for LADC exemplified by use of Tyrosine kinase inhibitors (TKIs) developed to target mutant components of the receptor tyrosine kinase (RTK) pathways such as EGFR, ALK and ROS1, frequently altered in LADC. ALK inhibitors such as crizotinib, ceritinib, alectinib and brigatinib, are effective against LADC tumors harboring ALK fusions and some ROS1-positive tumors to homology between the kinase domains of ROS1 and ALK. The types of molecular alterations in LSCC is often different from LADC and therefore other anticancer agents are effective. Example 4 provides data on therapy eligibility of NSCLC patients according to a LSCC or LADC diagnosis based on *KRT6ABC* expression.

In a further embodiment, a determined expression level of *PD-L1* above said reference level is indicative of a cancer subtype being susceptible to immunotherapy; whereas an expression level of *PD-L1*, equal to or below said reference level is indicative of a cancer subtype not being susceptible to immunotherapy. Immunotherapy eligibility of NSCLC patients is currently based on immunohistochemical determination of PD-L1 expression using tumor biopsy material. Example 5 provides data on immunotherapy eligibility of NSCLC patients based on *PD-L1* expression.

In an additional embodiment, determination of immunotherapy eligibility for a given cancer patient also can be performed for cancers beyond NSCLC.

In yet another embodiment, a determined expression level of *EGFR* above said reference level is indicative of a NSCLC subtype where EGFR-TKI's, such as gefitinib, erlotinib, afatinib, dacomitinib and osimertinib, is a treatment option; whereas an expression level of *EGFR* below said reference level is indicative of a NSCLC subtype where other agents are treatment options. Example 6 provides data on therapy eligibility of NSCLC patients based on *EGFR* expression.

In an embodiment, for NSCLC patients harbouring an *EGFR*-mutation and currently EGFR-TKI treated, a determined expression level of *VIM*, *ZEB1*, and *FGFR1* above said reference level and an expression level of *CDH1*, *EPCAM*, *ESRP1*, *and GRHL2* equal to or below said reference level is indicative of occurrence of EMT and resulting acquired or intrinsic resistance towards EGFR-TKI treatment. Such occurrence of EMT is indicative of a NSCLC subtype where other types of RTK-TKI's, chemotherapy or immunotherapy instead is a treatment option. Example 7 provides data on therapy eligibility of NSCLC patients for treatment based on deduction of EMT based on *VIM*, *ZEB1*, *FGFR1*, *CDH1*, *EPCAM*, *ESRP1*, *and GRHL2* expression.

In another embodiment, a determined expression level of *hTERT* above said reference level is indicative of cancer irrespective of cancer subtype; whereas an expression level of *hTERT*, equal to or below said reference level is indicative of absence of cancer in the given patient, or the cancer burden being below detection in the given patient at the current time, or the cancer being a low-grade cancer in the given patient. A screening result pointing on elevated *hTERT* expression level will allow an early intervention against malignant cancers of all types with health beneficial consequences. Example 8 provides data on screening for presence of cancer based on *hTERT* expression.

In yet another embodiment, a determined expression level of *KRT5* and/or *TP63* above said reference level is indicative of the NSCLC subtype being LSCC; a determined expression level of *NAPSA* and/or *NKX2-1* above said reference level is indicative of the NSCLC subtype being LADC; and a determined expression level of *INSIM1* above said reference level is indicative of the LC subtype being SCLC. Different treatment procedures exist according to the LC patient diagnosis being LSCC or LADC (example 4). Example 9 provides data on therapy eligibility of NSCLC patients according to a LSCC, LADC, or SCLC diagnosis based on *KRT5*, *TP63*, *NAPSA*, *NKX2-1*, and *INSIM1* expression.

In an embodiment, the individual is a mammal. In a preferred embodiment, the individual is a human.

In an embodiment, a treatment protocol is devised based on the assessment of the method.

In an embodiment, the subject is already undergoing treatment for said cancer or has undergone treatment at the time of the sample being taken.

In another embodiment, a comparison of results from sample obtained previous in time is compared to results from a sample obtained later in time. In an embodiment, a treatment protocol has been initiated or completed between the sampling of the two samples. Thus, the sample obtained first may serve as a reference for the sample obtained later in time. Such analysis may indicate whether a cancer is progressing, regressing or is unchanged.

In another embodiment, a treatment regime is initiated based on the identified subtype of a cancer, stage of a cancer and/or risk of developing cancer.

### Kit of parts

The technology of the present invention could also be foreseen to be incorporated into a kit. Thus, an aspect of the invention relates a kit of parts comprising
- a first container comprising an antibody (or other binding moiety) against a histone modification, said histone being modified indicative of being associated with an expressed or repressed gene;
- a second container comprising one or more, preferably two primers, for a gene of interest, preferably the gene is a *KRT6* gene, more preferably *KRT6ABC*; and
- optionally, one or more further containers comprising primer sets for one or more further genes of interest, such as one or more genes functioning as positive or negative controls for gene expression;
- optionally, one or more containers comprising components for initiating a PCR reaction using said primers;
- optionally one or more probes for detecting a PCR product; and
- optionally instructions for using the kit in a method according to the invention.

Probes for detecting PCT products are e.g. relevant if ddPCR is used. See e.g. example 4, wherein ddPCR is used for subtyping lung cancers.

The one or more further containers comprising primer sets for one or more further genes of interest, such as one or more genes functioning as positive or negative controls for gene expression, could comprise primers for ACTG1, ALK and/or SAT2. In example 2, it is verified that these genes may function as positive and negative controls.

The one or more containers comprising components for initiating a PCR reaction using said primers; could comprise polymerase and/or dNTPs etc.

### Uses

The present invention may have different uses. In an aspect, the invention relates to the use of a kit according to the invention for determining a subtype of a cancer, staging a cancer, and/or a risk of developing cancer.

In another aspect, the invention relates to the use of genes or part of genes associated with nucleosomes comprising histones, said histones being indicative of being associated with an expressed gene or a repressed gene for determining a subtype of a cancer, staging a cancer, and/or a risk of developing cancer.

It should be noted that embodiments and features described in the context of one of the aspects of the present invention also apply to the other aspects of the invention.

All patent and non-patent references cited in the present application, are hereby incorporated by reference in their entirety.

The invention will now be described in further details in the following non-limiting examples.

### Examples

### Example 1 - rationale of the cell-free Chromatin Immunoprecipitation (cfChIP) method

The following example describes the rationale of the cell-free Chromatin Immunoprecipitation (cfChIP) method and the individual steps of the procedure. The DNA of transcribed genes have specific histone modifications according to the transcriptional level. The histone modification H3K36me3 is located over the gene bodies of actively transcribed regions, and the occupancy is generally directly correlated to gene transcription and thus expression, whereas other histone modifications are located over inactive genes (e.g. H3K9me3) (Figure 1A). This relationship constitutes the rationale behind the here described method: cell-free Chromatin Immunoprecipitation (cfChIP). This method measures the occupancy of specific histone modifications over specific genes and utilize this relationship to infer the transcriptional level and thus gene expression. Due to the higher presence of circulating nucleosomes in plasma of cancer patients this method is designed to measure gene specific occupancy of H3K36me3 in the plasma of cancer patients to infer gene expression in the tumor, which otherwise usually requires a tissue biopsy from the tumor.

The procedure of the methods consists of the following overall steps (illustrated in Figure 1B:
1) Cell-free DNA is extracted and purified from a blood sample (plasma) aliquot (or another type of body liquid) to serve as a reference (referred to as an input sample) and ideally contains equal amounts of all cell-free DNA sequences present in the sample.
2) Anti-H3K36me3 antibodies are coupled to magnetic beads and added to the remaining plasma sample which bind to all DNA sequences with H3K36me3 contained in form of nucleosomes/histones (i.e. present over all active transcribed gene sequences)
3) Bead-Antibody-H3K36me3-DNA complexes are captured and pulled down using a magnet
4) Captured complexes are denatured and enriched DNA is extracted and purified (referred to as an IP sample)
5) DNA sequences corresponding to specific genes are quantified in Input and IP samples using sensitive quantitative PCR methods such as, but not limited to, ddPCR, real-time quantitative PCR (qPCR), next generation sequencing, and Nanostring nEncounter. The transcriptional activity of genes of interest can then be evaluated through comparisons of known active and inactive gene sequences in input and IP samples.

### Example 2 - Proof of method of the invention

### Aim of study

To examine the correlations between H3K36me3 enrichment and gene expression and to validate central aspects and reagents of the protocol by performing in vitro experiments on 4 different *in vitro* grown NSCLC cell lines (PC9, HCC827, A549, and H1666).

### Materials and methods

### Cell culture

PC9, HCC827, and H1666 cells (purchased from ATCC) were grown in RPMI supplemented with 10 % fetal bovine serum and 1 % Penicillin-streptomycin (Gibco, Thermo Fischer Scientific, Waltham, MA, USA). A549 cells (purchased from ATCC) were grown in DMEM supplemented with 10 % fetal bovine serum and 1 % Penicillin-streptomycin (Gibco, Thermo Fischer Scientific, Waltham, MA, USA). The cells were grown at 37°C and 5 % CO₂.

### Chromatin Immunoprecipitation (ChIP)

Immunoprecipitations were performed on chromatin from PC9, HCC827, A549, and H1666 cells. Chromatin was prepared from cells grown in dishes to confluence by crosslinking in media containing 1% formaldehyde for 10 minutes at room temperature. The crosslinking was quenched by the addition of 125 mM glycine and 5 minutes incubation at room temperature. Following washing with ice-cold PBS, cells were collected by spinning at 1000*g* for 5 min at 4°C and lysed in 50 µL ChIP lysis buffer per 10^6 cells. Lysates were fragmented by sonication (5 min cycles of pulses for 30s on, 30s? off), to an average fragment length of 200-500 bp, adding ice to the waterbath between each round. For immunoprecipitation, 25µL pre-washed protein A/G magnetic beads were incubated with either anti-H3K36me3 (Abcam, ab9050) or rabbit IgG (Invitrogen, 100005291) antibody and incubated for 1 hour at 4°C with rotation. Antibody-bead complexes were blocked in RIPA buffer containing 1% BSA and incubated for 30 mins. at 4°C. Blocked antibody-bead complexes were added to 12µg of chromatin and incubated overnight with rotation at 4°C. Bead-bound antigen/antibody complexes were collected and washed using a dynamag followed by elution in TE buffer containing 1% SDS for 1 hour at 65°C. Following bead removal samples were treated with 40µg Proteinase K for additional 2 hours at 65°C. Eluted DNA was extracted using phenol: chloroform.

### Quantitative PCR (qPCR)

qPCR and measurements were run in duplicate reactions of 10 µL each containing 0.125 µL forward primer (10 pmol/µL), 0.125 µL reverse primer (10 pmol/µL), 3.750 µL nuclease-free water, 5 µL SYBR green (Roche, Bassel, Switzerland) and 1 µL DNA or cDNA. Analyses were performed on a Roche Lightcycler 480 with the following settings: heating at 95°C for 15 min, 45 cycles of PCR (95°C 10 sec, 60°C 20 sec, 72°C 15 sec) and final elongation at 72°C for 1 min. Measurements were performed using the following primers:

| | qPCR primers (ChIP) | |
|---|---|---|
| **SEQ ID NO:** | **Name** | **Sequence (5' - 3')** |
| 1 | ACTG1 fw | GCT GTT CCA GGC TCT GTT CC |
| 2 | ACTG1 rw | GCT CAC ACG CCA CAA CAT G |
| 3 | ALK fw | CAG CAT AGG CCA AGT ACA CG |
| 4 | ALK rw | TAT TTT CTT CCA GCC CCA GG |
| 5 | SAT2 fw | TCA TCC AAC GGA AGC TAA TG |
| 6 | SAT2 rw | CGT TTC AAT TCG ATG GTG TT |

### Results

Using H3K36me3 specific antibodies or non-specific IgG antibodies we performed ChIP on chromatin prepared from four NSCLC cell lines: PC9, HCC827, A549, and H1666. Using qPCR the enrichment was determined over the following specific gene loci:
1) A transcribed region of the gene *ACTG1* which is a constitutively expressed gene in all tissues and cells (frequently used as a positive control for H3K36me3 ChIP experiments);
2) A transcribed region of *ALK* which is transcriptionally silenced gene in most tissues; and
3) The repetitive pericentric DNA satellite sequence *SAT2*, which is condensed in heterochromatin devoid of H3K36me3 (frequently used as a negative control for H3K36me3 ChIP experiments).

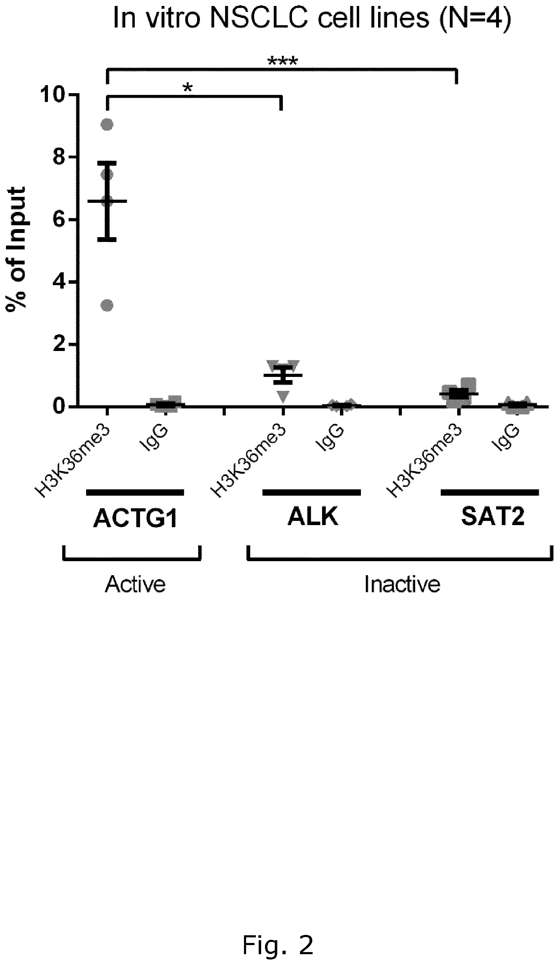

From *in silico* analysis using the GTEx database, *ACTG1* was found to be highly expressed in most tissues, whereas *ALK* were found to be very low expressed. In agreement with these observations, we found *ACTG1* to be higher enriched compared to *ALK* and *SAT2* in the H3K36me3 immunoprecipitated sample (see figure 2), suggesting a positive correlation between H3K36me3 enrichment and gene expression. For all three loci IgG immunoprecipitations showed enrichment close to the limit of detection, indicating a very low background signal from non-specific antibody binding.

### Conclusion

In conclusion, this example validates the overall procedures, antibody, and reagents of the ChIP protocol and provides proof of the positive correlation between H3K36me3 enrichment and gene expression.

### Example 3 - Proof of concept

### Aim of study

To utilize cell-free ChIP (cfChIP) to first proof the presence of circulating nucleosomes/histones carrying native H3K36me histone modifications. Second, proof the quantification of measured H3K36me3 in circulating nucleosomes/histones can infer the gene expression of the associated DNA corresponding to the three genetic loci validated *in vitro* in example 1.

### Materials and methods

### Plasma samples

Peripheral blood was collected into EDTA-containing tube. Samples were processed within 2 hours by centrifugation at 1400g for 15 min at room temperature. Plasma was isolated and aliquots were stored at -80°C until further use.

### Cell-free Chromatin Immunoprecipitation (cfChIP)

Plasma samples stored at -80°C was thawed on ice and spun at 16.000g for 10 min. at 4°C to remove cellular debris. 400µL plasma was saved for input and extracted by QIAmp Circulating Nucleic Acid kit (Qiagen, 55114). Plasma (3-6.5 mL) were diluted 5 times in RIPA buffer and precleared by incubation with 25µL prewashed magnetic protein A/G beads for a minimum of 2 hours to capture unspecific binding albumin proteins and plasma antibodies. Meanwhile, 20µL washed beads were incubated with either anti-H3K36me3 (Abcam, ab9050) or rabbit IgG (Invitrogen, 100005291) antibody and incubated for 1 hour at 4°C with rotation. Antibody-bead complexes were blocked in RIPA buffer containing 0.2 ng/µL salmon sperm DNA and 1% BSA and incubated for 30 mins. at 4°C. Preclearing beads were removed from plasma using a dynamag magnet and incubated with the blocked antibody-bead complexes overnight at 4°C with rotation. Antibody-bound complexes were collected and washed twice in low salt buffer, twice in high salt buffer, and once in TE buffer. Complexes were eluted from beads in two fractions by adding elution buffer, incubated at 65°C for 1 hour each, and subsequently pooled. Lastly, eluted DNA was extracted using phenol : chloroform.

### ddPCR

The ddPCR reactions were performed using the QX200 AutoDG Droplet Digital PCR System (Bio-Rad). Duplex measurements were run in triplicate reactions of 20 µL each containing 11 µL 2X ddPCR Supermix for Probes (no UTP, Bio-Rad), 1 µL of each primer-probe assay, 1 µL nuclease-free water, and 7 µL cfDNA. Droplets were prepared using the QX200 AutoDG (BioRad). PCR was performed on a GeneAmp PCR System 9700 instrument (Applied Biosystems). Droplets were analyzed on a QX200 Droplet Reader (BioRad). Results were obtained and analyzed as recommended by the manufacturer using QuantaSoft Software version 1.7.4. The threshold for positive droplets were set using analyzed droplets from non-template control measurements. Measurements were performed using the following ddPCR primers and probes:

| **SEQ ID NO:** | **Name** | **Sequence 5'-3'** |
|---|---|---|
| 7 | ACTG1 dd fw | GTT TCT TTC GCT GTT CCA |
| 8 | ACTG1 dd rw | GCA GGC AGA AAC CAA AT |
| 9 | ACTG1 dd pr | HEX-CCC GGC ATT TCC TCC CTG AAG CCT CC-BHQ1 |
| | ALK | Bio-Rad PrimePCR ddPCR Expression Probe Assay: dHsaCPE5040668 (FAM) |

An alternative setup used the following ddPCR primers and probes showed the same overall results.

| **SEQ ID NO:** | **Name** | **Sequence 5'-3'** |
|---|---|---|
| 17 | ALK Ex27 F | TGT GGG TGG GTG TGT CTA TA |
| 18 | ALK Ex27 R | ATT TCC CAT AGC AGC ACT CC |
| 19 | ALK Ex27 pr | FAM-TGT CCT CTG TCC CAT GCC CAG GTC CT-BHQ1 |

### Results

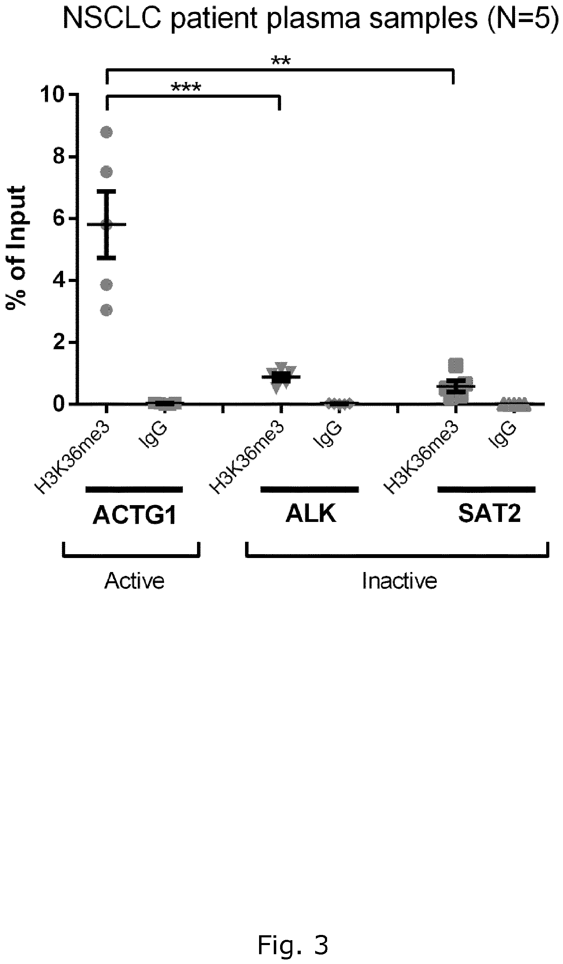

cfChIP was performed on plasma from five patients with advanced stage NSCLC using either anti-H3K36me3 or unspecific IgG antibodies. As a proof-of-concept, levels of H3K36me3 were quantified over the *in vitro* verified *ACTG1* and *ALK* loci using ddPCR. As observed *in vitro*, the mean enrichment in relation to input samples was found to be 6.6-fold higher enriched for *ACTG1* than *ALK* in the plasma IP samples of the NSCLC patients (see figure 3). The enrichment at both *ACTG1* and *ALK* for the IgG samples were at borderline for the limit of detection (0.03% and 0.02% respectively), and both were significantly lower than the H3K36me3 IP samples, demonstrating a very low contribution of background signal in the IP samples. In addition, *SAT2* enrichment was quantified by qPCR. Owing to the highly repetitive nature of *SAT2*, accurate measurements can be obtained in samples with very low concentrations of DNA by the lesser sensitive qPCR, even when using tiny amounts of sample compared to ddPCR. The enrichment of the *SAT2* locus was comparable to *ALK* in the IP samples and IgG samples showed similar depletion as *ACTG1* and *ALK* (see figure 3). Compared to the substantial inter individual difference in concentration of cfDNA in the plasma, the difference in enrichment relative to input of all three genes were similar. This indicates a reliable and robust normalization for this method, as well as a wide dynamic range for the requirements to sample cfDNA concentration.

### Conclusion

Given the universally transcriptional activity of *ACTG1* and the higher enrichment of H3K36me3 compared to the universally transcriptional inactivity of *ALK* and *SAT2,* these results collectively demonstrate proof-of-concept for cfChIP as a method of inferring gene expression from a blood sample.

### Example 4 - NSCLC patient subtyping using cfChIP inferred gene expression

### Aim of study

To utilize cfChIP as an indirect measure of gene expression for *in silico* identified and *in vitro* validated cancer subtype specific genes, to effectively distinguish between lung adenocarcinoma (LADC) and lung squamous cell cancer (LSCC) patients using blood plasma. Since, no known DNA mutations can readily distinguish between LADC and LSCC, the cancer subtype is for the time being usually determined by immunohistochemical analysis on tissue biopsies.

### Materials and methods

For in vitro validation, Cell culture, RNA extraction and cDNA synthesis, ChIP, and qPCR was performed as described in example 1, with the addition of the following primers for qPCR:

| | qPCR primers (ChIP): | |
|---|---|---|
| **SEQ ID NO:** | **Name** | **Sequence (5' - 3')** |
| 10 | KRT6ABC (ChIP) fw | CTG AGG CTG AGT CCT GGT A |
| 11 | KRT6ABC (ChIP) rw | AAG TCT GCA GTC CTC TG |

### RNA extraction and cDNA synthesis

RNA extraction was performed using TRI Reagent according to manufacturer's instructions (Sigma-Aldrich, St. Louis, MO, USA). cDNA was prepared in 20 µL reactions using the iScriptTM cDNA Synthesis Kit according to the manufacturer's instructions (Bio-Rad, Hercules, CA, USA). Synthesized cDNA was diluted 5 times in nuclease-free water.

### Reverse transcriptase quantitative PCR (RT-qPCR)

RT-qPCR measurements were run in duplicate reactions of 10 µL each containing 0.125 µL forward primer (10 pmol/µL), 0.125 µL reverse primer (10 pmol/µL), 3.750 µL nuclease-free water, 5 µL SYBR green (Roche, Bassel, Switzerland) and 1 µL DNA or cDNA. Analyses were performed on a Roche Lightcycler 480 with the following settings: heating at 95°C for 15 min, 45 cycles of PCR (95°C 10 sec, 60°C 20 sec, 72°C 15 sec) and final elongation at 72°C for 1 min. Measurements were detected using the following primers:

| | RT-qPCR primers (mRNA) | |
|---|---|---|
| **SEQ ID NO:** | **Name** | **Sequence (5' - 3')** |
| 12 | KRT6ABC fw | CTG AGG TCA AGG CCC AAT |
| 13 | KRT6ABC rw | CGG TGG ATC TCA GCA ATC TC |

For in vivo characterization, handling of plasma samples, cfChIP, and ddPCR was performed as described in example 2, with the addition of the following primers and probes for ddPCR:

| | ddPCR primers and probes | |
|---|---|---|
| **SEQ ID NO:** | **Name** | **Sequence 5'-3'** |
| 14 | KRT6ABC dd fw | CTG AGG CTG AGT CCT GGT A |
| 15 | KRT6ABC dd rw | AAG TCT GCA GTC CTC TG |
| 16 | KRT6ABC dd pr | FAM-AGC AGG GAG TGG GCA GCC GCT-BHQ1 |

### Results

First an *in silico* analysis of the gene expression profile of LADC and LSCC using the TCGA Wanderer database on known IHC specific biomarkers was conducted. It was found that the *KRT6* isoforms *A, B,* and *C* (in the following the three *KRT6* isoforms in common abbreviated *KRT6ABC*) are specifically upregulated in LSCC compared to LADC.

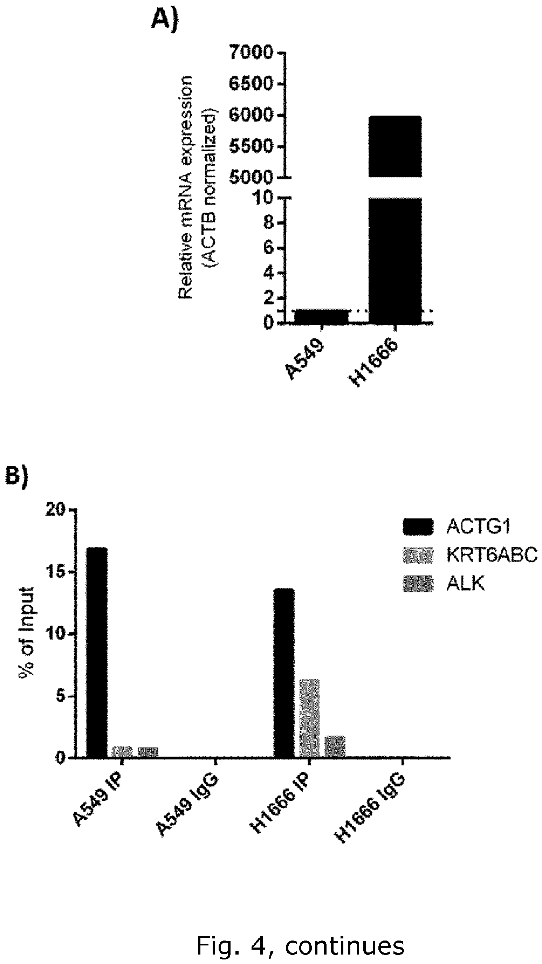

Second, whether the correlation between H3K36me3 enrichment and mRNA expression is also evident for the *KRT6ABC* loci was experimental demonstrated. RT-qPCR analysis on H1666 and A549 cells revealed a higher expression in H1666 than A549 cells (see figure 4A). Owing to the high sequence similarity, a single primer set to collectively amplify all three isoforms was designed and used. This difference was also observed from ChIP analyses in the H3K36me3 enrichment at the *KRT6ABC* loci revealed by qPCR, also using a single primer set to detect all three loci, denoted *KRT6ABC* (see figure 4B).

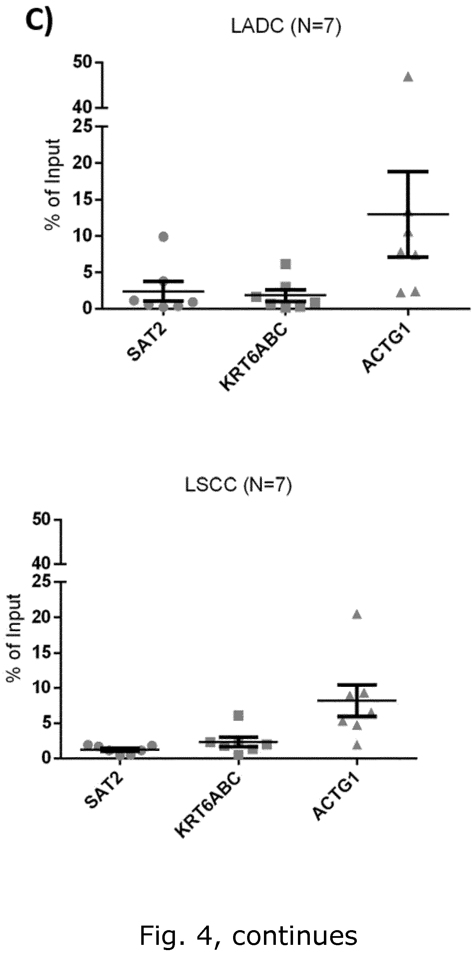

Finally, cfChIP was performed on plasma from 14 NSCLC patients (LADC; N=7, LSCC; N=7). Using ddPCR analysis, *ACTG1* and *KRT6ABC* DNA was quantified by duplex measurements in IP and input samples. *SAT2* was measured by qPCR. Compared to *KRT6ABC* and *SAT2*, *ACTG1* was higher enriched in both LADC and LSCC samples, demonstrating a successful immunoprecipitation. Moreover, the mean enrichment of *KRT6ABC* in LSCC was 1.9-fold higher than *SAT2,* whereas in LADC enrichment of *KRT6ABC* was lower than *SAT2* (0.76-fold) (see figure 4C). These results indicate a higher presence of H3K36me3 at the *KRT6ABC* in LSCC compared to LADC, and hence a higher expression, which is in agreement with previous observations.

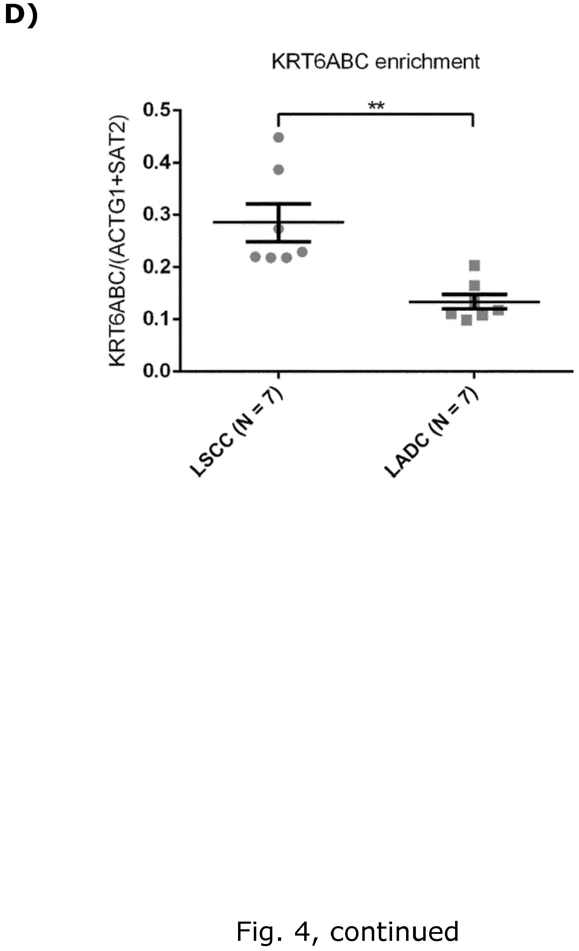

To further investigate this difference, we normalized the enrichment of KRT6ABC to ACTG1 or SAT2 for each patient analogous to the normalization to multiple references genes in qPCR by geometric means. A significant higher KRT6ABC enrichment in LSCC patients compared to LADC to a magnitude of 2.1-fold was found (see figure 4D).

### Conclusion

Collectively these results show a higher occupancy of H3K36me3 at the *KRT6ABC* isoform loci in LSCC compared to LADC.

*In vitro* examination determined a positive correlation of H3K36me3 and mRNA expression at the *KRT6ABC* loci.

Hence, a higher H3K36me3 occupancy infers a higher *KRT6ABC* expression and effectively demonstrate cfChIP as a diagnostic tool to indirectly infer the gene expression level of the said gene in the tumor, and successfully distinguish between LADC and LSCC patients. This distinction has until now only been possible by analyses from tumor biopsy material.

### Example 5 - Immunotherapy eligibility of NSCLC patients based on cfChIP inferred PD-L1 expression (hypothetical example)

### Aim of study

Today NSCLC patients are offered immunotherapy if more than 50% of tumor cell exhibit PD-L1 (also abbreviated CD274) expression. This measurement requires a tumor biopsy and is considered the golden-standard albeit having multiple shortcomings, the largest being heterogeneity of the tumor which will produce false negative and false positive measurements. This study aims to provide *in vitro* proof of correlation between H3K36me3 enrichment and gene expression for PD-L1 to utilize cfChIP to infer the expression of *PD-L1* gene in the tumor from plasma isolated from a blood sample. Cell-free tumor nucleosomes and DNA is allegedly less prone to false measurements due to tumor heterogeneity. If successful this will provide a non-invasive means of evaluating eligibility of immunotherapy of possible higher sensitivity and specificity.

### Materials and methods

### Cell culture

HCC827, (purchased from ATCC) were grown in RPMI supplemented with 10 % fetal bovine serum and 1 % Penicillin-streptomycin (Gibco, Thermo Fischer Scientific, Waltham, MA, USA). Erlotinib resistant HCC827 cells, denoted HCC827 ER (established previously in our lab) were grown in the presence of 5µM erlotinib. The cells were grown at 37°C and 5 % CO₂

### RNA sequencing and differential expression analysis

RNA extraction was performed using TRI Reagent according to manufacturer's instructions (Sigma-Aldrich, St. Louis, MO, USA). Subsequent library construction, sequencing, post-sequencing adaptor removal as well as initial filtering steps were performed by BGI. 100 bp paired-end (PE) sequencing was performed on an Illumina HiSeq platform. Approximately 40 million clean PE reads were produced per sample. Transcript quantification from reads was performed using SALMON r package. Differential expression analysis was performed between HCC827 cell and HCC827 erlotinib resistant cells based on quantified transcript counts using DEseq2. Genes with more than a 2-fold change in expression and an adjusted *p* value < 0.05 were denoted as differentially expressed.

### Chromatin Immunoprecipitation (ChIP)

Immunoprecipitations were performed on chromatin prepared from HCC827 and HCC827 erlotinib resistant cells (HCC827 ER) as described in example 1

### ChIP-sequencing and differential binding analysis

Input and immunoprecipitated DNA samples from HCC827 and HCC827 ER cells were subjected to 50bp single end (SE) sequencing, a next generation sequencing (NGS) procedure. Library construction, sequencing, post-sequencing adaptor removal as well as initial filtering steps were performed by BGI. Sequencing was performed on a BGIseq500 platform to produce a minimum of 40 million clean reads per sample. Reads were mapped to the genome (hg19) using STAR.

Differential binding analysis and annotation was performed using DiffRep according to the authors' suggested default pipeline using a window size of 1000 bp with a step size of 100 bp. Significant differential peaks (p < 0.05) were annotated to genes using region analysis, as part of the DiffRep analysis

### Results

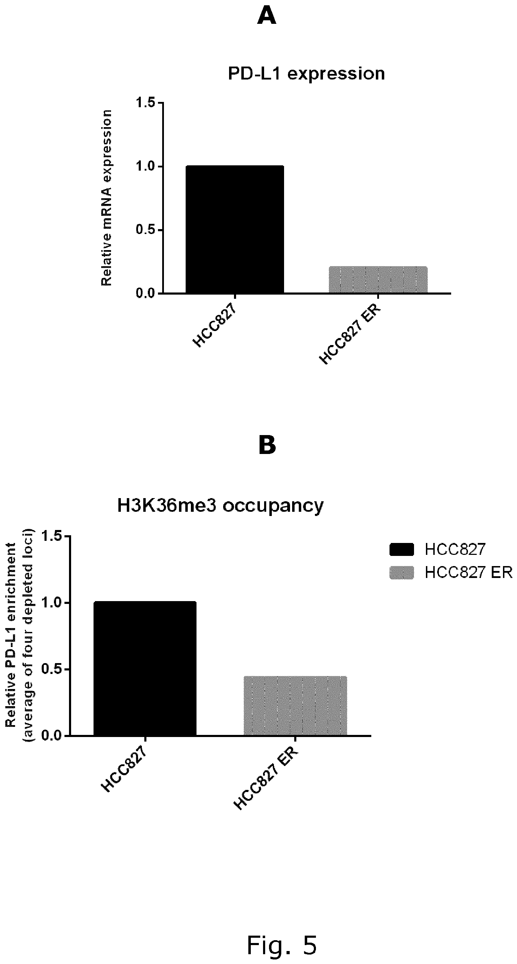

RNA-sequencing analysis performed on these cells revealed a statistically significant 5-fold lower expression of *PD-L1* in HCC827 ER cells compared to HCC827 cells (see Figure 5A). This downregulation was accompanied by a statistically significant depletion in H3K36me3 in four genetic loci corresponding to the *PD-L1* gene body (see figure 5B). Thus, these results suggest the positive correlation between H3K36me3 and gene expression is also evident for *PD-L1.*

Next, plasma samples collected from patients reported to have 100% PD-L1 positive cells and 0% PD-L1 positive cells will be evaluated by cfChIP for the positive inference of PD-L1 gene expression. In addition, plasma samples from patients becoming unresponsive to immunotherapy treatment will be evaluated by cfChIP, to determine the capabilities of monitoring PD-L1 expression and thus effectiveness of treatment, to allow for early intervention.

### Conclusion

H3K36me3 occupancy over the *PD-L1* gene body reflects the gene transcription and expression. It should be obvious to those skilled in the arts that based on these results, *PD-L1* serves as a gene of interest in the utilization of cfChIP for the improved diagnosis and prognosis of NSCLC patients in evaluating the eligibility for immunotherapy.

### Example 6 - EGFR-TKI eligibility of EGFR wildtype NSCLC patients based on cfChIP inferred expression of EGFR (hypothetical example)

### Aim of study

Some wildtype-EGFR NSCLC patients respond well to EGFR tyrosine kinase inhibitors, and evidence suggests this to be because of a marked upregulation of wildtype EGFR. In addition, EGFR-mutated NSCLC patients inevitably develop resistance to EGFR-TKI, which is evidenced to be accompanied by an EGFR downregulation. Thus, monitoring EGFR expression in these patients will likely provide means of early detection of resistance development and allow for earlier intervention of treatment. To identify these patients for improved treatment, this study aims to provide *in vitro* proof of the correlation between H3K36me3 enrichment and gene expression for EGFR. This correlation will enable the expression of EGFR in the tumor to be inferred by utilization of cfChIP measurements from plasma isolated from a blood sample.

### Materials and methods

All materials and methods are as described in example 4.

### Results

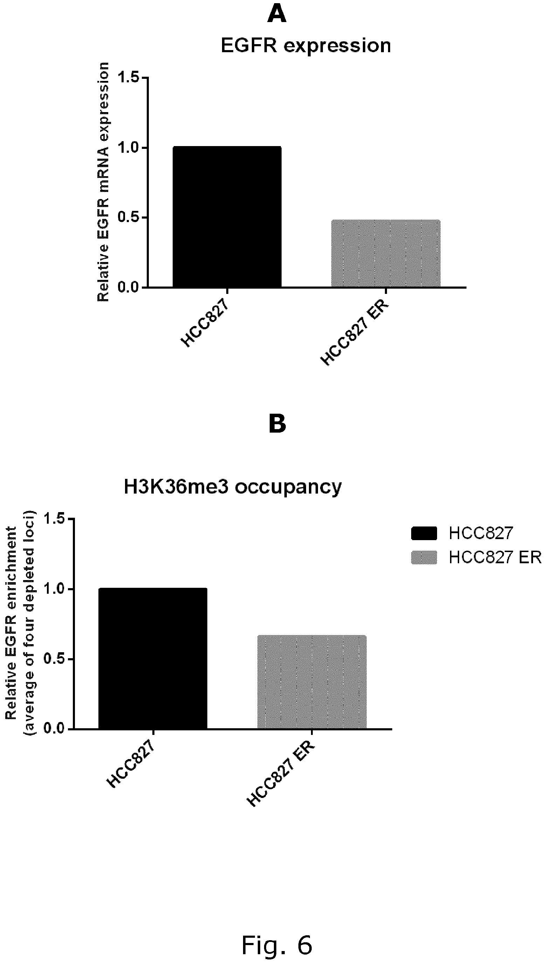

RNA-sequencing analysis performed on these cells (described in example 4) revealed a statistically significant 2-fold lower expression of *EGFR* in HCC827 ER cells compared to HCC827 cells (see figure 6A). This downregulation was accompanied by a statistically significant depletion in H3K36me3 averaged over four significant genetic loci corresponding to the *EGFR* gene body (see Figure 6B). Thus, these results suggest the positive correlation between H3K36me3 and gene expression is also evident for *EGFR.*

Next, plasma samples collected from EGFR wildtype patients reported to respond to EGFR-TKI treatment will be evaluated by cfChIP for the positive inference of *EGFR* gene expression compared to patients unresponsive to EGFR treatment. Similarly, plasma samples will be evaluated by cfChIP from EGFR-TKI resistant patients with known resistance mechanism unrelated to EGFR to evaluate the capability of detecting the onset of resistance.

### Conclusion

H3K36me3 occupancy over the *EGFR* gene body reflects the gene transcription and expression. It should be obvious to those skilled in the arts that based on these results *EGFR* serves as a gene of interest in the utilization of cfChIP for the improved treatment and monitoring of EGFR-TKI resistance in NSCLC patients.

### Example 7 - Detection of Epithelial-Mesenchymal Transition (EMT) in NSCLC patients based on cfChIP inferred expression of EMT markers (hypothetical example).

### Aim of study

An increasingly recognized resistance mechanism of multiple treatments in NSCLC is the process of epithelial-mesenchymal transition (EMT). EMT is a transcriptional program of phenotypic plasticity repolarizing epithelial cells towards a mesenchymal phenotype. Molecularly EMT is characterized by the downregulation of epithelial marker genes including, but not limited to, *CDH1*, *EPCAM*, *ESRP1*, and *GRHL2,* and an upregulation of mesenchymal marker genes including, but not limited to, *VIM*, *ZEB1*, and *FGFR1.* Today, EMT is not part of the post-diagnostic workup of relapsed NSCLC patients, since detection requires a tissue biopsy and is therefore primarily detected post mortem. To this end, this study aims to provide *in vitro* proof of the correlation between H3K36me3 enrichment and gene expression for selected EMT markers. This correlation will enable the expression of EMT markers in the tumor to be inferred by utilization of cfChIP measurements from plasma isolated from a blood sample.

### Materials and methods

### All materials and methods are as described in example 4

### Results

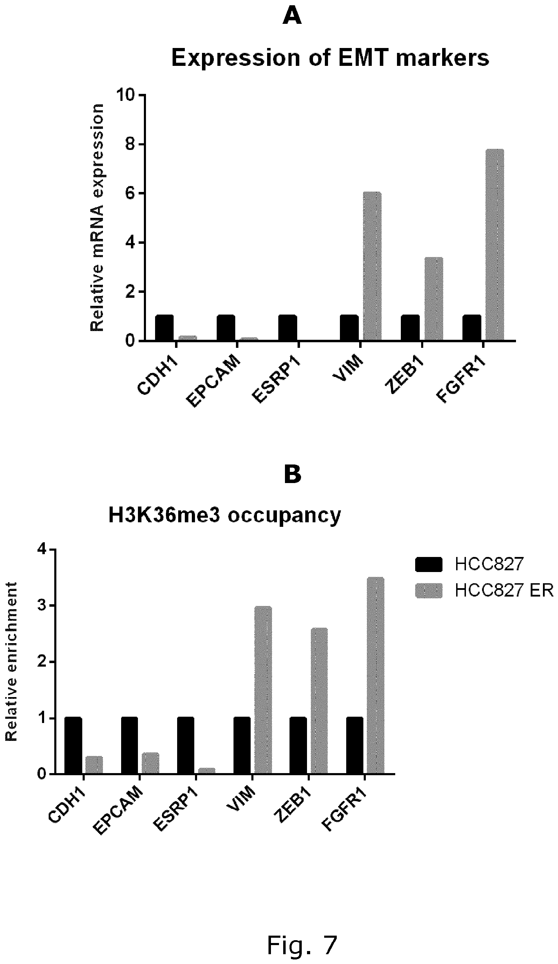

RNA-sequencing analysis performed on these cells (described in example 4) revealed a statistically significant lower expression of the epithelial markers *CDH1*, *EPCAM*, *ESRP1*, and *GRHL2* in HCC827 ER cells compared to HCC827. This downregulation was accompanied by a statistically significant depletion of H3K36me3 over the genetic loci corresponding to these gene bodies (see Figure 7). In contrast, mRNA expression of the mesenchymal markers *VIM*, *ZEB1*, and *FGFR1* was significantly upregulated in HCC827 ER cells compared to HCC827, which was accompanied by an enrichment of sequences corresponding to the genetic loci of these gene bodies (see Figure 7). EGFR in HCC827 ER cells compared to HCC827 cells. This downregulation was accompanied by a statistically significant depletion in H3K36me3 averaged over four significant genetic loci corresponding to the gene bodies (transcribed part of the genes).

Thus, these results suggest the positive correlation between H3K36me3 and gene expression is also evident for EMT marker genes.

### Conclusion

H3K36me3 occupancy over these selected EMT genes reflects the gene transcription and expression. It should be obvious to those skilled in the arts that based on these results, these EMT markers serves as genes of interest in the utilization of cfChIP for the detection of EMT in relapsed NSCLC patients.

### Example 8 - Screening of patients for cancer based on cfChIP inferred expression of hTERT (hypothetical example).

### Aim of study

As an early event during carcinogenesis, many cancers become reliant on the reactivation of the otherwise inactivated telomerase gene *hTERT* for continued proliferation. Thus, routine evaluation of *hTERT* expression would constitute a potential diagnostic screening procedure for cancer. To this end this study would aim to provide proof of the correlation between H3K36me3 enrichment and gene expression of *hTERT* expression. Such correlation would be evaluated for the capability of effectively distinguish diagnosed cancer patient from healthy individuals based on cfChIP inferred expression of *hTERT* from blood plasma.

### Materials and methods

### All materials and methods are as described in example 1 and 2

### Results

Positive correlation of H3K36me3 and gene expression will be prooved based on material from cell lines with differential expression of *hTERT.* Next, plasma samples collected from cancer patients with identified promoter mutations in *hTERT* gene in the tumor (which significantly increases the transcription of *hTERT*) will be evaluated by cfChIP for the positive inference of gene expression compared to healthy individuals. Results will be evaluated for predictive capabilities to be used for diagnostic and screening procedures.

### Conclusion

Positive correlation between H3K36me3 and *hTERT* gene expression will enable *hTERT* as a potential target of interest interest in the utilization of cfChIP. Successful distinction between healthy individuals and diagnosed cancer patient using cfChIP inferred *hTERT* expression would constitute a diagnostic screening procedure for cancer.

### Example 9 - Improved capabilities of lung cancer subtyping with inclusion of additional lung squamous cell carcinoma (LSCC) specific markers as well as markers specific for lung adenocarcinoma (LADC) (hypothetical example)

### Aims of study

Current diagnostic procedures for characterizing and subtyping lung cancer relies on multiple immunohistochemical markers besides the in here described LSCC specific *KRT6ABC* markers. These markers include the additional LSCC markers *KRT5* and *TP63*, the LADC specific markers *NAPSA* and *NKX2-1,* as well as the small cell lung cancer (SCLC) marker *INSM1.* Therefore, to improve on the current diagnostic potential of cfChIP in lung cancer, this study aims to provide proof of the correlation between H3K36me3 enrichment and gene expression of these markers to act as effective genes of interest to be utilized by cfChIP for the improved diagnostic capabilities of the current assay described in experiments 1-7. This will provide the ability to successfully and accurately diagnose the following types of lung cancer: SCLC, LADC (NSCLC), and LSCC (NSCLC).

### Materials and methods

All materials and methods are as described in examples above

### Results

Positive correlation of H3K36me3 and gene expression will be proved based on material from cell lines with differential expression of the following genes: *NAPSA, NKX2-1*, *TP63*, and *INSM1.* Next, plasma samples collected from lung cancer patients diagnosed with SCLC, LADC (NSCLC), or LSCC (NSCLC) will be evaluated by cfChIP for the positive inference of gene expression among the three cancer types. Results will be evaluated for the successful capabilities to accurately distinguish between the lung cancer subtypes be used as a blood based diagnostic tool.

### Conclusion

Successful distinction between SCLC, LADC (NSCLC), or LSCC (NSCLC) diagnosed cancer patient using cfChIP inferred expression of *NAPSA*, *NKX2-1*, *TP63*, and *INSM1* together with the cfChIP inferred expression described in examples 1-7 will constitute a classification panel for lung cancer diagnostics.

## Claims

1. A method of subtyping a cancer, staging a cancer, or determining the risk of developing cancer for an individual, said method comprising the steps of
a) contacting a biological sample from said individual with an antibody or antibody fragment that binds to a nucleosome and/or histone;
b) isolating nucleosomes and/or histones associated to said antibody;
c) optionally, purifying DNA associated with said nucleosomes and/or histones;
d) identifying and/or quantifying at least one gene or part of gene associated with the isolated nucleosome and/or histone or present in the optionally purified DNA, to identify and/or quantify the level of expression of a gene in said sample from said individual;
e) optionally, comparing said identified and/or quantified expression level of the at least one gene to one or more reference levels; and
f) determining a subtype of a cancer, staging a cancer, and/or a risk of developing cancer for said individual, based on the identification/quantification of the expression level of the one or more genes.

2. The method according to claim 1, wherein the gene is selected from the group consisting of a KRT6 gene, such as *KRT6A*, *KRT6B*, *and KRT6C*, *ACTG1*, *ALK*, *SAT2*, *EGFR*, *hTERT*, *PD-L1*, *FGFR1*, *CDH1*, *VIM*, *ZEB1*, *KRT5*,*TP63*, *INSM1*, *NAPSA*, and *NKX2-1* or combinations thereof, preferably *KRT6A*, *KRT6B*, and *KRT6C.*

3. The method according to claim 1 or 2, wherein the gene is a *KRT6* gene such as *KRT6A, KRT6B* and/or *KRT6C* or combinations thereof, such as *KRT6ABC.*

4. The method according to any of the preceding claims, wherein the cancer is selected from the group consisting of lung cancer, such as Non-small-cell lung carcinoma (NSCLC), such as adenocarcinoma (LADC), squamous cell carcinoma (LSCC), and large cell carcinoma (LCC) and small cell lung cancer (SCLC).

5. The method according to any of the preceding claims, wherein the subtyping and/or staging of a cancer is subtyping or staging LADC and LSCC.

6. The method according to any of the preceding claims, wherein level of *ACTG1, ALK* and/or *SAT2* is also determined.

7. The method according to any of the preceding claims, wherein a determined expression level of a *KRT6* gene, preferably *KRT6ABC,* above said reference level is indicative of a lung cancer being a LSCC; whereas an expression level of a *KRT6* gene, preferably *KRT6ABC,* equal to or below said reference level, is indicative of a lung cancer being a LADC.

8. The method according to any of claim 1-6, wherein a determined expression level of *PD-L1* above said reference level is indicative of a cancer subtype being susceptible to immunotherapy; whereas an expression level of *PD-L1,* equal to or below said reference level is indicative of a cancer subtype not being susceptible to immunotherapy.

9. The method according to any of the preceding claims, wherein said biological sample is selected from the group consisting of a blood sample, such as whole blood, blood plasma or blood serum, saliva, urine, CSF or a tissue sample, preferably a blood plasma sample.

10. The method according to any of the preceding claims, wherein said biological sample is a blood sample, preferably a blood plasma sample.

11. The method according to any of the preceding claims, wherein the histone is a histone or modified histone considered to be associated with an expressed gene or a repressed gene, such as a constitutively expressed gene or a cell type specific expressed gene, or a constitutively repressed gene, or a cell type specific repressed gene, such as the histone being selected from the group consisting of H3K36me3, H3K36me2 and H3K36mel, preferably H3K36me3.

12. The method according to any of the preceding claims, wherein the histone is H3K36me3.

13. A kit of parts comprising
• a first container comprising an antibody (or other binding moiety) against a histone modification, said histone being modified indicative of being associated with an expressed or repressed gene;
• a second container comprising one or more, preferably two primers, for a gene of interest, preferably the gene is a *KRT6* gene, more preferably *KRT6ABC;* and
• optionally, one or more further containers comprising primer sets for one or more further genes of interest, such as one or more genes functioning as positive or negative controls for gene expression;
• optionally, one or more containers comprising components for initiating a PCR reaction using the said primers; and
• optionally one or more probes for detecting a PCR product;
• optionally instructions for using the kit in a method according to any of claims 1-12.

14. Use of a kit according to claim 13, for determining a subtype of a cancer, staging a cancer, and/or a risk of developing cancer.

15. Use of genes or part of genes associated with nucleosomes comprising histones, said histones being indicative of being associated with an expressed or repressed gene for determining a subtype of a cancer, staging a cancer, and/or a risk of developing cancer.
